# EUROPEAN PATENT APPLICATION

(11) **EP 2 783 726 A1**
(43) Date of publication of application: **01.10.2014**
(21) Application number: 14161938.7
(22) Date of filing: 27.03.2014
(51) Int. Cl.: A61N 1/05, A61N 1/36, A61B 18/14

(54) **Stimulating electrode, particularly for electrical stimulation treatments**

(30) Priority: 28.03.2013 IT MO20130080
(71) Applicant: Neurimpulse S.r.l., 35030 Rubano (PD) (IT)
(72) Inventor: Reverberi, Claudio, 41037 Mirandola (MO) (IT); Sluijter, Menno Emanuel, 6064 OW Kerns (NL)
(74) Representative: Modiano, Micaela Nadia

(57) **Abstract**

A stimulating electrode (1), particularly for electrical stimulation treatments, comprising a catheter (2) for supporting a first stimulation pole (3) adapted to be placed in direct contact with a portion of tissue (100) of the patient to be treated and to be coupled electrically to at least one second stimulation pole (4), the first and second poles being supplied with voltages of opposite sign by an electrical stimulator for the flow of electrical charges through the portion of tissue (100) to be treated that is interposed between the poles.

The peculiarity of the invention consists in that the at least one second pole (4) is associated with the catheter (2) and is adapted to make contact directly with the portion of tissue (100) to be treated, proximate to the first pole (3).

## Description

The present invention relates to a stimulating electrode, particularly for electrical stimulation treatments.

The use is known of stimulating electrodes that are temporarily placed proximate to the region to be stimulated for the treatment of chronic pain. Such technique is used for the stimulation of nervous tissue, of tissue of the joints, such as for example joints of the shoulder, of the knee or of the thigh, and for the treatment of intervertebral discogenic pain and of the peripheral nervous system, particularly for the stimulation of nervous ganglia in order to relieve pain. Such methodology requires a pulsed electrical stimulation of the tissue to be treated through the use of a monopolar electrode in direct contact with the tissue to be stimulated. By way of puncturing the skin, the operator positions the electrode in contact with the region to be treated using an adapted peripheral venous catheter with a body made of insulating material, and, simultaneously, externally arranges a conductive plate with an ample surface in direct contact with the outer portion of the skin of the patient, proximate to the tissue to be treated. After such positioning, the operator powers both the electrode and the conductive plate by way of an external electrical stimulator, usually called a radiofrequency current generator, with electrical voltages of opposite sign. Such voltage of opposite signs enables the passage of electrical charges through the portion of the body of the patient which is interposed between the two poles, which acts as an electrical conductor, thus enabling the stimulation of the tissue to be treated.

Such conventional treatment is not devoid of drawbacks, including the fact that it prevents a complete selective stimulation of the portion of tissue to be treated, permitting only the stimulation of a wide region interposed between the two poles. In fact, the passage of electrical charges from the tip of the electrode to a point of the conductive plate cannot be defined in advance, in that the portion of the body which is interposed between the two poles contains different types of tissue and locally varying thicknesses, offering a greater or lesser resistance to the passage of the charges and causing the flow of electrons to deviate in directions that are difficult to predict.

Another drawback of such conventional treatment consists in that it does not permit control of the quantity of electrical charges, i.e. of the intensity of the current, supplied solely for the stimulation of the tissue to be treated, in that part of the electrical charges used are dispersed in the body of the patient. In order to ensure a correct treatment of the tissue with an adequate level of current for the stimulation, the operator supplies a higher level of current than what is theoretically sufficient for the stimulation, so as to compensate for such dispersion. However, supplying a level of current that is too high increases the risk of damaging the stimulated tissue, in that a concentration of electrical charges at a single point, especially proximate to the tip of the electrode in contact with the stimulated tissue, will progressively increase the local temperature which, if higher than a certain threshold, will irremediably damage the stimulated nerve or tissue. In order to be able to monitor the local temperature and prevent damage, the electrode is provided with a thermocouple feedback for temperature control.

A further drawback of such conventional treatment consists in that it requires a lengthy application time, consequently increasing the surgical operating time and the risk of damaging the stimulated tissue. In fact, the operator, not knowing in advance the level of current necessary for the stimulation alone, begins the treatment with a certain level of current and increases it progressively, monitoring the local temperature by way of adapted signaling means associated with the thermocouple located on the tip of the electrode. Such methodology prolongs the treatment for a longer time than that theoretically necessary, which ensures an adequate stimulation of the painful part, but also increases the risk of damaging the tissue.

The aim of the present invention consists in providing a stimulating electrode, particularly for electrical stimulation treatments, that eliminates the drawbacks and overcomes the limitations of the known art by enabling the selective stimulation of only the portion of tissue to be treated, thus avoiding the stimulation of adjacent portions of tissue.

Within this aim, an object of the present invention is to provide an electrode that makes it possible to supply the right quantity of electrical charges only in the region to be treated, thus avoiding electrical dispersions in the body.

Another object of the invention consists in providing an electrode that is capable of reducing treatment times with respect to conventional electrodes, thus reducing the risk of causing damage.

Another object of the invention consists of providing an electrode that is capable of offering the widest guarantees of reliability and safety in use.

Another object of the invention consists in providing an electrode that is easy to implement and economically competitive when compared to the known art.

This aim and these and other objects which will become better apparent hereinafter are achieved by a stimulating electrode, particularly for electrical stimulation treatments, comprising a catheter for supporting a first stimulation pole adapted to be placed in direct contact with a portion of tissue of the patient to be treated and to be coupled electrically to at least one second stimulation pole, the first and second poles being supplied with voltages of opposite sign by an electrical stimulator for the flow of electrical charges through the portion of tissue to be treated that is interposed between said poles, characterized in that said at least one second pole is associated with said catheter and is adapted to make contact directly with the portion of tissue to be treated proximate to said first pole.

Further characteristics and advantages of the invention will become better apparent from the detailed description of a preferred, but not exclusive, embodiment of a stimulating electrode, particularly for electrical stimulation treatments, which is illustrated for the purposes of non-limiting example with the assistance of the accompanying drawings wherein:
Figure 1 is a schematic sectional view taken along a longitudinal plane of a stimulating electrode, particularly for electrical stimulation treatments, according to the invention, which is located proximate to the tissue to be treated and with a first pole and a second pole for stimulation in the inactive configuration;
Figure 2 is a schematic view of the electrode in Figure 1 with the first pole and the second pole in the active configuration.

With reference to the figures, the stimulating electrode, particularly for electrical stimulation treatments, generally designated by the reference numeral 1, comprises a catheter 2 for supporting a first stimulation pole 3 that is adapted to be placed in direct contact with a portion of tissue 100 of the patient to be treated and coupled electrically to at least one second stimulation pole 4.

According to the invention, the second pole 4 is also associated with the catheter 2 and is adapted to make contact directly with the portion of tissue 100, proximate to the first pole 3. The first pole 3 and the second pole 4 are supplied with electrical voltages of opposite sign by way of a conventional electrical stimulator, usually referred to in the trade as a radiofrequency current generator and not shown in the accompanying figures, so as to generate a passing of electrical charges between the poles through the portion of tissue 100 which, consequently, is electrically stimulated.

The catheter 2 can be for example of the peripheral venous catheter type for electrical stimulation treatments and has a cylindrical body that is provided with two longitudinal channels 6 in which the first pole 3 and the second pole 4 respectively can slide.

In the description that follows the terms "distal" and "proximal" are used with reference to the position of the operator during the use of the electrode according to the invention.

On a distal portion 2a of the catheter 2 there are two exit holes 5 of the respective channels 6. The sliding of the poles 3 and 4 inside the respective channels 6 enables the transition between an inactive configuration, in which the poles 3 and 4 are retracted inside the catheter 2, and an active configuration, in which the end portions of the poles protrude from the respective exit holes 5 which themselves protrude from the electrode 2. Conveniently, the exit holes 5 are positioned on diametrically opposite surfaces of the distal portion 2a. The transition between the inactive configuration and the active configuration is carried out by the operator by way of adapted means 7 for moving the first pole 3 and the second pole 4, which are arranged at the proximal portion 2b of the catheter 2.

Conveniently, each one of the channels 6 has a respective end portion 6a, proximate to the respective exit hole 5, which extends along a direction 8 that is substantially transverse to the longitudinal axis 11 of the catheter 2. The directions 8 of extension of the two channels 6 mutually diverge, so as to guide the end portions of the poles 3 and 4 to mutually move apart during the exit thereof through the respective exit holes 5.

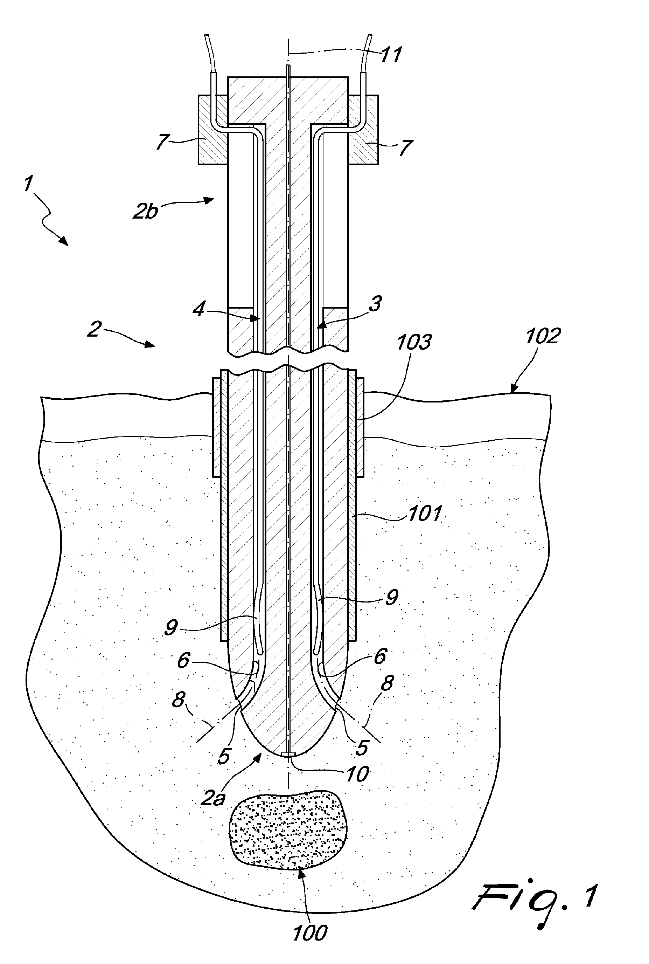

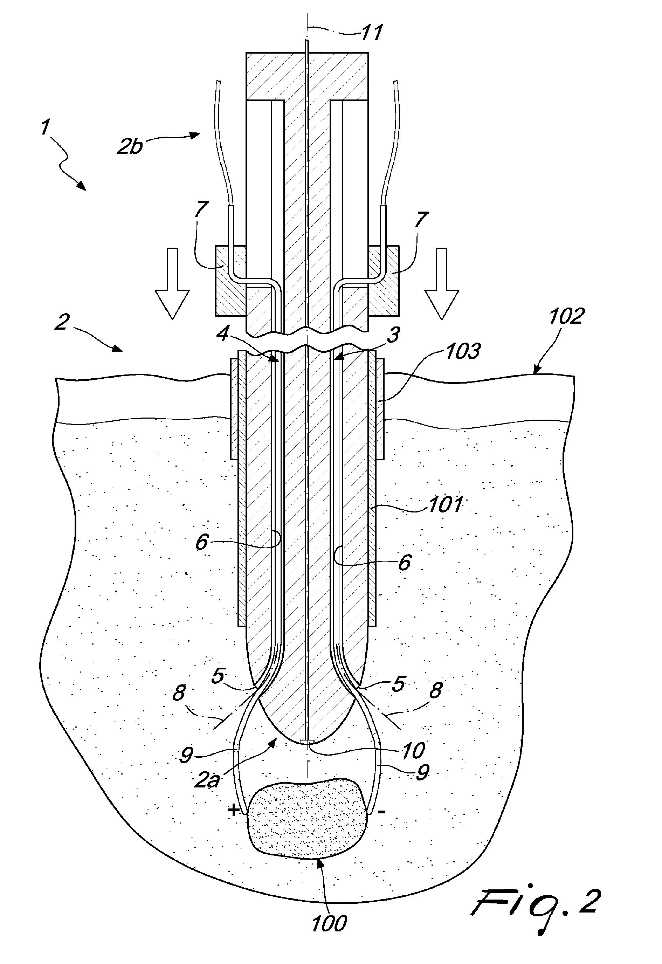

Conveniently, both the first pole 3 and the second pole 4 have a substantially wire-like body that is sufficiently rigid that it can be actuated by the movement means 7 and slide easily inside the respective channel 6. Furthermore, each pole 3 and 4 is provided with a contact tip portion 9, which protrudes from the exit holes 5 in the active configuration, and comprises an arc-like portion. The arc-like portions of the poles 3 and 4 have their respective inside curves facing each other in the active configuration, as illustrated in Figure 2, so as to be able to move apart during the transition from the inactive configuration to the active configuration and be able to embrace points of the portion of tissue 100 to be stimulated which are spaced apart, or more conveniently, opposite ends of a nervous ganglion. Advantageously, the contact tip portions 9 are made of electrically conductive and elastically deformable material that can, thus, deform elastically in order to re-enter the channel 6 in the inactive configuration, as illustrated in Figure 1, and resume their curvilinear shape in the active configuration upon each application, as illustrated in Figure 2. Particularly, in the active configuration, the free ends of the contact tip portions 9 are mutually spaced by an extent that is sufficiently wide to prevent the electrical charges from jumping between the two poles only without passing through the portion of tissue 100 to be stimulated, and is sufficiently narrow that the electrical charges will still be attracted to the other pole, without which condition the stimulation of the portion of tissue would be prevented. The distance between the poles 3 and 4 is dependent on the voltage supplied by the electrical stimulator and is substantially comprised between 0.015 meters and 0.025 meters, and is preferably 0.02 meters for an electrical voltage of approximately 25 volts. In order to prevent short-circuits between the poles 3 and 4 and enable the correct positioning proximate to the tissue to be stimulated, the catheter 2 is made of flexible, insulating material and is provided with an atraumatic profile on the outer surface of the distal portion 2a. Furthermore, the catheter 2 has an outer space occupation that is dimensioned so as to be able to slide inside a guide body 101 which can be positioned under the skin 102 proximate to the tissue 100 to be treated; in turn the guide body 101 can be inserted in an outer jacket 103 that can be used to puncture the initial layers of the skin 102 together with a metallic needle, according to known methods. Furthermore, the catheter 2 comprises a thermocouple 10, at the distal portion 2a thereof, which can be associated with signaling means, not shown, for monitoring the temperature reached by the stimulated portion of tissue 100 during the treatment.

In other embodiments, not shown in the accompanying figures, the poles 3 and 4 can have different geometric shapes and each one of the contact tip portions 9 can comprise at least one rectilinear portion that is angularly distanced from the longitudinal axis 11. Furthermore, the electrode 1 can comprise more than two stimulation poles, of which at least one is supplied with electrical voltage of the opposite sign with respect to the electrical voltage of the other poles in order to enable the electrical charges to pass between them. Multiple poles can be slideable in one of the at least two channels 6 and be supplied with the same voltage or each pole can be slideable in a respective channel that is defined inside the catheter 2. Furthermore, in other embodiments, the thermocouple 10 can also be accommodated slidingly within a respective channel and be movable between an inactive configuration, in which it is substantially retracted inside the distal portion 2a, and an active configuration, in which it is protruding from the respective channel in order to come into contact with the portion of tissue 100 being subjected to stimulation and monitor, by direct contact, the temperature of that tissue.

Operation of the stimulating electrode, particularly for electrical stimulation treatments, is described below.

The operator positions the electrode 1 proximate to the portion of tissue 100 to be treated, substantially following conventional medical procedures. In particular, the operator first uses a metallic needle covered by the outer jacket 103 to puncture the skin 102 and the muscular fascia. Subsequently, the operator removes the needle only and inserts the guide body 101 together with a mandrel inside it into the outer jacket 103. The operator, by conveniently moving the mandrel, places the guide body 101 proximate to the portion of tissue 100 to be treated. The operator concludes the positioning by removing the mandrel and replacing it with the electrode 1 with the poles 3 and 4 in the inactive configuration. Finally, the operator, by acting on the proximal portion 2b outside the body of the patient, can further correct the positioning of the catheter 2, by moving it along a direction that is substantially coaxial to the longitudinal axis 11 in order to move closer to or farther from, for example by a few centimeters, the portion of tissue 100 to be treated, or by rotating the catheter about its longitudinal axis 11 in order to be able to conveniently orient the subsequent exit of the two poles 3 and 4. Following the correct positioning of the catheter 2, the operator then acts on the movement means 7 that are arranged on the proximal portion 2b, thus enabling the transition from the inactive configuration to the active configuration and allowing the exit of the end portions of the poles 3 and 4 through the respective exit holes 5. During such transition the contact tip portions 9, thanks to the elastic properties of the material of which they are made and to the geometry of the end portions 6a, exit from the respective exit holes 5, reacquiring their curved shape, and move away from the distal portion 2a and move away from each other up to a distance that, in contact with the articulation, the intervertebral disc or the ganglion to be stimulated, is substantially comprised between 0.015 meters and 0.025 meters. Once the contact tip portions 9 are positioned in direct contact with the portion of tissue 100 to be treated, the operator supplies the two poles 3 and 4 with electrical voltages of opposite signs, thus beginning the electrical stimulation. During the treatment, the electrical charges pass from the first pole 3 to the second pole 4, or vice versa (according to the polarization), passing through the interposed portion of tissue 100 only. During the treatment, the operator can vary the voltage or the intensity of current supplied by way of the electrical stimulator. Once the treatment is finished, the operator retracts the contact tip portions 9 inside the distal portion 2a by actuating the movement means 7, thus passing from the active configuration to the inactive configuration.

In practice it has been found that the stimulating electrode, particularly for electrical stimulation treatments, according to the present invention, achieves the intended aim and objects in that it makes it possible to selectively stimulate only the portion of tissue to be treated, while preventing dispersions of electrical charges in the adjacent portions of tissue. In fact, the exchange of electrical charges that are adapted to stimulate the tissue occurs exclusively between the two poles which, being positioned in direct contact with the portion of tissue to be treated, limit the area of travel of the electrical charges, thus preventing the charges from being dispersed in adjacent portions of tissue or in other nearby tissues.

Another advantage of the electrode, according to the invention, consists in that it supplies a well-defined quantity of electrical charges that are exclusively useful for the stimulation of the portion of tissue to be treated, thus avoiding supplying greater quantities of electrical charges that could damage the tissue.

Another advantage of the electrode, according to the invention, consists in that it reduces treatment times with respect to the application times of conventional treatments. In fact, the selective supply of a determined quantity of charges makes it possible to optimize the treatment and reduce the application times. Furthermore, a treatment with reduced times reduces any risk of overheating of the treated tissue, thus making electrostimulation more reliable.

The stimulating electrode, particularly for electrical stimulation treatments, thus conceived is susceptible of numerous modifications and variations, all of which are within the scope of the appended claims.

Moreover, all the details may be substituted by other, technically equivalent elements.

In practice the materials employed, provided they are compatible with the specific use, and the contingent dimensions and shapes, may be any according to requirements.

The content of Italian patent application no. MO2013A000080, the priority of which is claimed in the present application, is incorporated as a reference.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. A stimulating electrode (1), particularly for electrical stimulation treatments, comprising a catheter (2) for supporting a first stimulation pole (3) adapted to be placed in direct contact with a portion of tissue (100) of the patient to be treated and to be coupled electrically to at least one second stimulation pole (4), the first and second poles (3, 4) being supplied with voltages of opposite sign by an electrical stimulator for the flow of electrical charges through the portion of tissue (100) to be treated that is interposed between said poles, **characterized in that** said at least one second pole (4) is associated with said catheter (2) and is adapted to make contact directly with the portion of tissue (100) to be treated proximate to said first pole (3).

2. The electrode (1) according to claim 1, **characterized in that** said first pole (3) and said at least one second pole (4) have a linear shape and are inserted slidingly within respective longitudinal channels (6) that are extended along said catheter (2) for transition between an inactive configuration, in which said first pole (3) and said at least one second pole (4) are retracted inside said catheter, and an active configuration, in which end portions of said first pole (3) and said at least one second pole (4) exit from respective exit holes (5) of said channels (6) which are provided on a distal portion (2a) of said catheter (2) for direct contact with the portion of tissue (100) to be treated.

3. The electrode (1) according to claim 2, **characterized in that** said exit holes (5) are arranged on diametrically opposite surfaces of said distal portion (2a).

4. The electrode (1) according to one or more of the preceding claims, **characterized in that** it comprises means (7) for moving said first pole (3) and said at least one second pole (4) along the respective channels (6), said movement means (7) being arranged at the proximal portion (2b) of said catheter (2) and being actuatable by an operator for the transition of said poles (3, 4) between said inactive configuration and said active configuration.

5. The electrode (1) according to one or more of the preceding claims, **characterized in that** said channels (6) have respective end portions (6a), proximate to the corresponding exit holes (5), that are extended along directions (8) that substantially mutually diverge.

6. The electrode (1) according to one or more of the preceding claims, **characterized in that** each one of said first pole (3) and at least one second pole (4) has a substantially wire-like body provided with a contact tip portion (9), which protrudes at least partly from said exit holes (5) in said active configuration, and comprises an arc-like portion, said arc-like portions being arranged so that their inside curves face each other.

7. The electrode (1) according to one or more of the preceding claims, **characterized in that** said contact tip portions (9) are made of electrically conducting and elastically deformable material.

8. The electrode (1) according to one or more of the preceding claims, **characterized in that** the free ends of said contact tip portions (9) in said active configuration are mutually spaced by an extent that is comprised substantially between 0.015 meters and 0.025 meters.

9. The electrode (1) according to one or more of the preceding claims, **characterized in that** it comprises a thermocouple (10), at said distal portion (2a), that can be associated with signaling means for monitoring the temperature reached by the stimulated portion of tissue (100) during the treatment.
